# FASCICULE DE BREVET EUROPEEN

(11) **EP 2 493 885 B1**
(45) Date de publication et mention de la délivrance du brevet: **18.12.2013**
(21) Numéro de dépôt: 10774188.6
(22) Date de dépôt: 28.10.2010
(51) Int. Cl.: C07D 407/06

(54) **MOLECULES POLYKETIDES COMME AGENTS ANTICANCEREUX**
POLYKETIDMOLEKÜLE ALS ANTIKREBSMITTEL
POLYKETIDE MOLECULES AS ANTICANCER AGENTS

(30) Priorité: 28.10.2009 FR 0957594
(43) Date de publication de la demande: 05.09.2012
(73) Titulaire: Pierre Fabre Médicament, 92100 Boulogne-Billancourt (FR); Centre National de la Recherche Scientifique (C.N.R.S.), 75016 Paris (FR); Institut De Recherche Pour Le Développement (IRD), 13000 Marseille (FR)
(72) Inventeur: CARLETTI, Isabelle, F-31400 Toulouse (FR); MASSIOT, Georges, F-51100 Reims (FR); DEBITUS, Cécile, Papeete 98713 (PF)
(74) Mandataire: Regimbeau
(86) Numéro de dépôt international: PCT/EP2010/066334
(87) Numéro de publication internationale: WO 2011/051380

(56) Documents cités:
- US-A1- 2003 018 013
- US-A1- 2005 004 185
- MUDIT M ET AL: "Discovery, design, and synthesis of anti-metastatic lead phenylmethylene hydantoins inspired by marine natural products", BIOORGANIC & MEDICINAL CHEMISTRY, PERGAMON, GB LNKD- DOI:10.1016/J.BMC.2008.12.053, vol. 17, no. 4, 15 février 2009 (2009-02-15), pages 1731-1738, XP025949583, ISSN: 0968-0896 [extrait le 2008-12-29]
- KASHMAN Y ET AL: "PTILOMYCALIN A: A NOVEL POLYCYCLIC GUANIDINE ALKALOID OF MARINE ORIGIN", JOURNAL OF THE AMERICAN CHEMICAL SOCIETY, AMERICAN CHEMICAL SOCIETY, NEW YORK, US LNKD- DOI:10.1021/JA00206A029, vol. 111, 1 janvier 1989 (1989-01-01), XP000942941, ISSN: 0002-7863

## Description

La présente invention concerne une molécule de type polykétide extraite d'un spongiaire et ses analogues, un procédé d'extraction et d'hémi-synthèse et l'utilisation de ces composés en tant que médicament, notamment pour le traitement du cancer.

De nombreux composés aux propriétés anticancéreuses sont décrits dans la littérature, par exemple des polykétides de type polyène linéaire isolés de cultures de *Streptomyces melanosporafaciens* et analogues proches (US 2005/004185) ou encore des composés de formule (I) suivante : 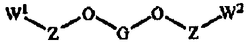 dans laquelle W¹ et W² peuvent représenter chacun un motif oxo-pyrane (US 2003/018013).

Depuis quelques dizaines d'années, les éponges marines sont devenues également le centre de nombreuses études depuis la mise en évidence de leur production de métabolites secondaires bioactifs, en particulier des alcaloïdes.

Kashman et al. (J. Am. Chem. Soc. 1989, 11, 8926-8928) décrit ainsi l'isolement d'un composé nommé ptilomycalin A des éponges marines *Ptilocaulis spiculifer* des Caraïbes et *Hermimycale sp.* de la mer rouge, ce composé possédant un motif de type guanidine polycyclique.

Mudit et al. (Bioorg. Med. Chem. 2009, 17(4), 1731-1738) décrit également l'isolement d'un dérivé de type hydantoïne de l'éponge marine *Hemimycale arabica* de la mer rouge et la préparation d'analogues.

Les inventeurs ont également pu isoler une nouvelle molécule de type polykétide à partir de l'éponge *Hemimycale sp.* qui présente une activité anticancéreuse.

La présente invention a donc pour objet un composé de formule (I) suivante : 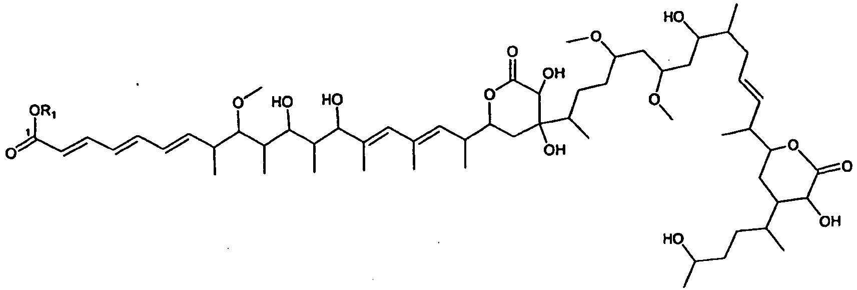 ou un sel pharmaceutiquement acceptable de celui-ci
pour laquelle R₁ représente un atome d'hydrogène ou un groupe (C₁-C₇)alkyle, par exemple un groupement (C₁-C₄)alkyle, par exemple un méthyle.

Par groupement « (C₁-C₇)alkyle », on entend, au sens de la présente invention, une chaine hydrocarbonée saturée, linéaire ou ramifiée, comportant de 1 à 7, par exemple de 1 à 4, et notamment 1, atomes de carbone. A titre d'exemple, on peut citer les groupes méthyle, éthyle, propyle, isopropyle, butyle, sec-butyle, tert-butyle, pentyle ou encore hexyle. Il pourra s'agir notamment d'un groupe méthyle.

Dans la présente invention, on entend désigner par «pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend désigner par « sels pharmaceutiquement acceptables » d'un composé, des sels qui sont pharmaceutiquement acceptables, comme défini ici, et qui possèdent l'activité pharmacologique souhaitée du composé parent. Il pourra s'agir plus particulièrement d'un sel d'addition de base. De tels sels sont formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique. Les bases organiques acceptables comprennent la diéthanolamine, l'éthanolamine, la N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Compte tenu du nombre d'atomes de carbone asymétriques présents sur cette molécule de formule (I), cette dernière peut prendre différentes configurations. Un composé selon l'invention pourra être en particulier un composé de structure défini par son mode d'extraction, notamment susceptible d'être obtenu par un procédé comprenant les étapes successives suivantes :
(i) macération d'un lyophilisat d'éponge *Hemimycale sp.* avec du chloroforme puis avec une solution hydro-éthanolique (notamment eau/éthanol 10/90), suivie d'une filtration pour donner un filtrat, puis concentration dudit filtrat pour donner un extrait,
(ii) partition de l'extrait obtenu à l'étape (i) précédente entre de l'eau et du dichlorométhane et séparation des phases aqueuse et organique résultantes, extraction de la phase aqueuse avec de l'acétate d'éthyle et séparation des nouvelles phases aqueuse et organique résultantes, réunion des deux phases organiques ainsi obtenues et concentration pour donner un extrait dessalé (les sels inorganiques ayant été éliminés dans la phase aqueuse),
(iii) reprise de l'extrait dessalé obtenu à l'étape (ii) précédente avec une solution hydro-méthanolique (notamment eau/méthanol 10/90) et de l'hexane, récupération et concentration de la phase méthanolique pour donner un extrait méthanolique,
(iv) isolation du composé de formule (I) avec R₁ = H à partir de l'extrait méthanolique obtenu à l'étape (iii) précédente, et
(v) éventuellement estérification de l'acide carboxylique en C-(1) pour donner un composé de formule (I) avec R₁ = (C₁-C₇)alkyle et/ou salification du composé obtenu à l'étape (iv) précédente, et isolation du composé ainsi obtenu du milieu réactionnel.

Dans le cadre de ce procédé, l'éponge *Hemimycale sp.* utilisée pourra être plus particulièrement originaire de l'archipel de Torres du Vanuatu.

Le composé de formule (I) pourra être choisi plus particulièrement parmi : 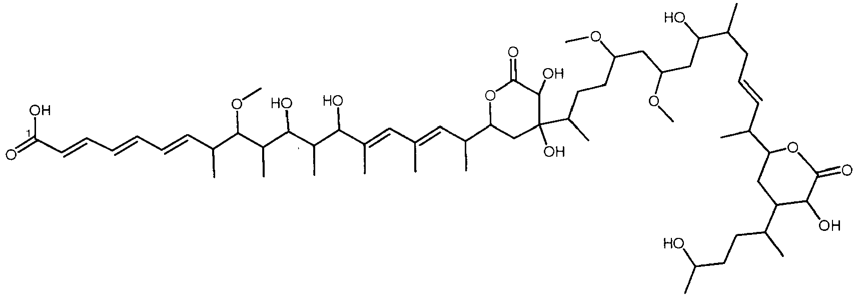 et

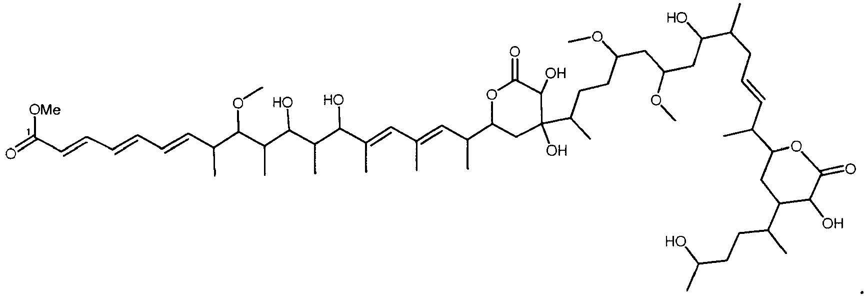

La présente invention a également pour objet un composé de formule (I) tel que défini ci-dessus pour son utilisation en tant que médicament, notamment destiné au traitement du cancer.

La présente invention concerne également l'utilisation d'un composé de formule (I) tel que décrit précédemment pour la préparation d'un médicament, destiné notamment au traitement du cancer.

L'invention concerne aussi une méthode de traitement d'une maladie proliférative, par exemple du cancer, comprenant l'administration à une personne en ayant besoin d'une dose efficace d'un composé de formule (I) tel que défini ci-dessus.

La présente invention a également pour objet une composition pharmaceutique comprenant au moins un composé de formule (I) tel que défini ci-dessus et un excipient pharmaceutiquement acceptable.

Les compositions pharmaceutiques de l'invention pourront être formulées par exemple pour une administration par voie intraveineuse ou orale.

Les composés de l'invention en tant que principes actifs peuvent être utilisés à des doses comprises entre 0,01 mg et 1000 mg par jour, par exemple en une seule dose.

Dans un mode de réalisation particulier de l'invention, la composition pharmaceutique est utilisée en tant que médicament, par exemple pour le traitement du cancer.

La présente invention a également pour objet un procédé de synthèse d'un composé tel que défini précédemment, comprenant les étapes successives suivantes :
(i) macération d'un lyophilisat d'éponge *Hemimycale sp.* avec du chloroforme puis avec une solution hydro-éthanolique (notamment eau/éthanol 10/90), suivie d'une filtration pour donner un filtrat, puis concentration dudit filtrat pour donner un extrait,
(ii) partition de l'extrait obtenu à l'étape (i) précédente entre de l'eau et du dichlorométhane et séparation des phases aqueuse et organique résultantes, extraction de la phase aqueuse avec de l'acétate d'éthyle et séparation des nouvelles phases aqueuse et organique résultantes, réunion des deux phases organiques ainsi obtenues et concentration pour donner un extrait dessalé,
(iii) reprise de l'extrait dessalé obtenu à l'étape (ii) précédente avec une solution hydro-méthanolique (notamment eau/méthanol 10/90) et de l'hexane, récupération et concentration de la phase méthanolique pour donner un extrait méthanolique,
(iv) isolation du composé de formule (I) avec R₁ = H à partir de l'extrait méthanolique obtenu à l'étape (iii) précédente, et
(v) éventuellement estérification de l'acide carboxylique en C-(1) pour donner un composé de formule (I) avec R₁ = (C₁-C₇)alkyle et/ou salification du composé obtenu à l'étape (iv) précédente, et isolation du composé ainsi obtenu du milieu réactionnel.

L'éponge utilisée dans ce procédé pourra être plus particulièrement originaire de l'archipel de Torres du Vanuatu.

L'isolation du composé selon l'invention avec R₁ = H (étape iv) pourra être réalisée notamment par une chromatographie sur gel de silice. Le produit obtenu pourra alors être purifié par des techniques bien connues de l'homme du métier, et notamment par chromatographie liquide haute performance (HPLC).

L'estérification de l'acide carboxylique à l'étape (v) pourra être réalisée par des protocoles bien connus de l'homme du métier. Dans le cas où l'on souhaite obtenir un ester méthylique, la réaction pourra être réalisée en présence de triméthylsilyldiazométhane (TMSCHN₂).

L'étape de salification de l'étape (v) pourra être réalisée par simple mélange du composé avec une base pharmaceutiquement acceptable, comme par exemple l'hydroxyde de sodium ou de potassium.

Le composé ainsi obtenu pourra être séparé du milieu réactionnel par des méthodes bien connues de l'homme du métier, comme par exemple par extraction, évaporation du solvant ou encore par précipitation et filtration.

Le composé pourra par ailleurs être purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

L'objet de la présente invention concerne également un procédé de synthèse d'un composé tel que défini précédemment avec R₁ représentant un groupe (C₁-C₇)alkyle à partir d'un composé de formule (I) tel que défini précédemment avec R₁ = H, comprenant les étapes successives suivantes :
(a) estérification de l'acide carboxylique en C-(1) pour donner un composé de formule (I) avec R₁ = (C₁-C₇)alkyle,
(b) éventuellement salification du composé obtenu à l'étape (a) précédente, et
(c) isolation du composé obtenu à l'étape (a) ou (b) précédente du milieu réactionnel.

### Etape (a) :

L'estérification de l'acide carboxylique pourra être réalisée par des protocoles bien connus de l'homme du métier. Dans le cas où l'on souhaite obtenir un ester méthylique, la réaction pourra être réalisée en présence de triméthylsilyldiazométhane (TMSCHN₂).

### Etape (b) :

L'étape de salification pourra être réalisée par simple mélange du composé avec une base pharmaceutiquement acceptable, comme par exemple l'hydroxyde de sodium ou de potassium.

### Etape (c) :

Le composé ainsi obtenu pourra être séparé du milieu réactionnel par des méthodes bien connues de l'homme du métier, comme par exemple par extraction, évaporation du solvant ou encore par précipitation et filtration.

Le composé pourra par ailleurs être purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

L'invention est plus particulièrement décrite de manière non limitative dans les exemples qui suivent.

### EXEMPLES

### Exemple 1 : Extraction du composé I-1 (composé de formule (I) avec R₁ = H)

5 kg frais d'éponge *Hemimycale sp.* ont été récoltés dans l'Archipel de Torres au Vanuatu puis lyophilisés pour donner un lyophilisat de 650g. Le lyophilisat a successivement été macéré pendant 6 heures avec du chloroforme puis avec une solution hydro-alcoolique (10:90 signifiant 10% d'eau et 90% d'éthanol). Cette seconde étape a été répétée deux fois. Les filtrats obtenus ont été regroupés, filtrés et concentrés à l'aide d'un évaporateur rotatif jusqu'à l'obtention d'un sirop aqueux. Ce dernier a successivement été partitionné avec du dichlorométhane et de l'acétate d'éthyle afin d'éliminer (dans la phase aqueuse) les sels inorganiques de l'extrait. L'extrait dessalé (42g) a été repris avec une solution hydro-méthanolique (10:90) et a été ensuite partitionnée avec de l'hexane pour éliminer les molécules les plus apolaires. Deux extraits, hexanique (24g) et méthanolique (18g) ont été ainsi obtenus. Seul l'extrait méthanolique a montré de l'activité pharmacologique.
Une méthode de suivi combinant à la fois test d'activité pharmacologique ainsi qu'analyse et purification à l'aide de la chromatographie couplée à la spectrométrie de masse (LC/MS) en mode électrospray négatif (ESI-) équipée d'un split préparatif a permis de mettre en évidence une famille de molécules avec les m/z 1061,8 ; 1059,4 ; 1077,7 ; 1093, 3 (+/-0.4 en ESI-) dont l'ion le plus significatif est m/z 1061,8.
Une chromatographie type « Flash » (ou « par étapes ») sur colonne de silice normale a été réalisée sur l'extrait méthanolique avec une élution en polarité croissante d'acétate d'éthyle et de méthanol afin obtenir 7 fractions. Les fractions N°4-7 qui ont montré de l'activité pharmacologique ont été ensuite chromatographiées sur colonne LH20 avec une élution de méthanol. Les purifications finales ont été réalisées par chromatographie liquide haute performance (HPLC) (RP C18) en utilisant un gradient d'eau et d'acétonitrile pour obtenir la molécule active de poids moléculaire 1062 (quantité 0,5mg, rendement 0.000077% par rapport au lyophilisat de l'éponge) ; HRESITOFMS m/z 1061,6780 (M-H)⁻, calculée pour C₅₉H₉₇O₁₆ m/z 1061.6782.
(HRESITOFMS = spectre de masse à temps de vol et à inonisation par électrospray haute résolution)

La molécule I-1 a ainsi été obtenue : 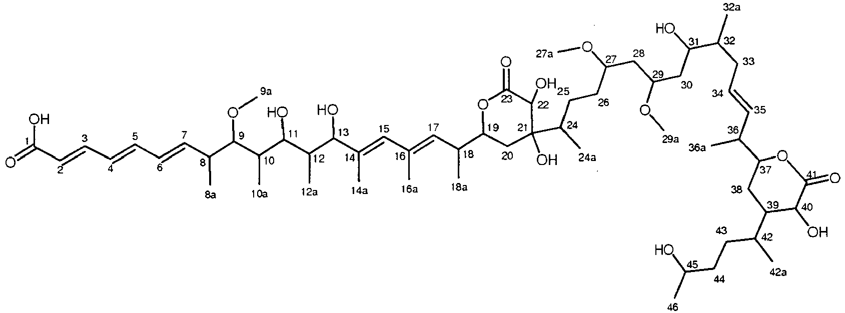 RMN ¹H (500MHz, METHANOL-d₄) δ = 7.10 (1H, dd, J = 15.1 Hz, J = 11.1 Hz, H-3), 6.45 (1H, dd, J = 15.0 Hz, J = 10.7 Hz, H-5), 6.27 (1H, dd, J = 15.0 Hz, J = 11.0 Hz, H-4), 6.17 (1H, dd, J = 15.3 Hz, J = 10.7 Hz, H-6), 5.95 (1H, br. s., H-15), 5.90 (1H, d, J = 15.3 Hz, H-2), 5.87 (1H, dd, J = 15.1 Hz, J = 8.7 Hz, H-7), 5.55 (1H, dt, J = 14.8 Hz, J = 7.3 Hz, H-34), 5.34 (1H, dd, J = 15.3 Hz, J = 8.5 Hz, H-35), 5.16 (1H, d, J = 9.8 Hz, H-17), 4.42 (1H, ddd, J = 11.3 Hz, J = 7.5 Hz, J = 3.5 Hz, H-19), 4.32 (1H, d, J = 11.3 Hz, H-40), 4.27 (1H, s, H-22), 4.11 (1H, ddd, J = 11.1 Hz, J = 7.7 Hz, J = 3.5 Hz, H-37), 3.99 (1H, d, J = 7.3 Hz, H-13), 3.66 - 3.74 (2H, m, H-31, 45), 3.59 - 3.66 (1H, m, H-29), 3.57 (1H, dd, J = 9.6 Hz, J = 2.0 Hz, H-11), 3.40 (3H, s, H-29a), 3.39 (3H, s, H-9a), 3.33 (3H, s, H-27a), 3.32-3.34 (1H, m, H-27), 3.27 (1H, dd, J = 6.7 Hz, J = 4.6 Hz, H-9), 2.74 - 2.87 (1H, m, H-18), 2.48 - 2.61 (1H, m, H-8), 2.29 - 2.39 (1H, m, H-36), 2.20 - 2.27 (1H, m, H-33), 2.02 - 1.22 (22H, m, H-10, 12, 20, 24, 25, 26, 28, 30, 32, 33, 38, 39, 42, 43, 44), 1.82 (3H, s, H-16a), 1.69 (3H, s, H-14a), 1.15 (3H, d, J = 6.4 Hz, H-46), 1.10 (3H, d, J = 6.7 Hz, H-18a), 1.11 (3H, d, J = 6.7 Hz, H-36a), 1.07 (3H, d, J = 6.7 Hz, H-8a), 0.98 - 1.02 (1H, m, H-25), 0.96 (3H, d, J = 6.7 Hz, H-24a), 0.93 (3H, d, H-12a), 0.91 (3H, d, J = 7.0 Hz, H-42a), 0.89 (3H, d, J = 6.7 Hz, H-32a), 0.85 (3H, d, J = 7.0 Hz, H-10a)
RMN ¹³C (126MHz, METHANOL-d₄) δ = 178.4 (C-41), 177.0 (C-23), 174.1 (C-1), 142.8 (C-7), 142.7 (C-3), 139.8 (C-5), 137.4 (C-14), 135.4 (C-16), 133.2 (C-34), 132.5 (C-35), 131.7 (C-15), 131.6 (C-17), 130.9 (C-6), 130.7 (C-4), 127.6 (C-2), 88.3 (C-9), 82.8 (C-19), 82.1 (C-13), 80.9 (C-37), 79.1 (C-27), 77.0 (C-29), 76.4 (C-21), 75.5 (C-11), 72.5 (C-22), 72.0 (C-31), 68.7 (C-45), 68.2 (C-40), 59.6 (C-9a), 57.9 (C-29a), 56.5 (C-27a), 43.6 (C-36), 41.5 (C-8), 41.4 (C-39), 40.8 (C-26), 40.8 (C-28), 40.6 (C-32), 40.1 (C-10), 39.3 (C-18), 39.3 (C-24), 38.5 (C-12), 38.0 (C-44), 37.7 (C-33), 33.7 (C-42), 33.0 (C-30), 32.2 (C-20), 32.1 (C-43), 28.0 (C-25), 26.7 (C-38), 23.6 (C-46), 17.9 (C-16a), 17.2 (C-36a), 17.2 (C-18a, 8a), 14.4 (C-32a), 14.0 (C-14a), 13.4 (C-42a), 13.1 (C-24a), 13.0 (C-10a), 7.6 (C-12a)

### Exemple 2 : Préparation de l'ester méthylique I-2 à partir de la molécule I-1

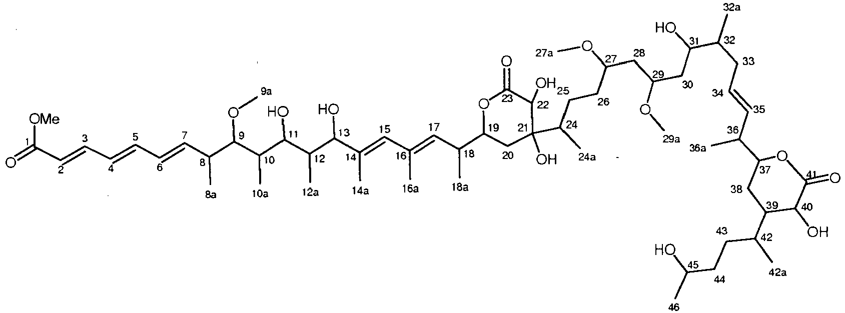

A une solution de 50µg de la molécule I-1 solubilisée dans 50µl de méthanol, a été ajoutée une goutte de triméthylsilyldiazométhane (TMSCHN₂). La réaction a été laissée sous agitation pendant 10 minutes à température ambiante pour être finalement séchée à l'aide d'un léger jet d'azote et analysée par LC/MS en mode ESI-. La réaction a été totale avec la formation de l'ester méthylique, moins polaire, de m/z 1075.4 (M-H)⁻.

### Exemple 3 : Inhibition de croissance d'un panel de lignées cellulaires cancéreuses humaines

Les cellules cancéreuses [lignées A549 (cancer du poumon non à petites cellules), BxPC3 (cancer du pancréas), LoVo (cancer du colon), MCF7 (cancer du sein), Namalwa (lymphome de Burkitt) et SK-OV-3 (cancer ovarien)] ont été ensemencées en plaque 96 puits dans du milieu RPMI 1640 sans rouge de phénol (Seromed) auquel est ajouté 10% de sérum de veau foetal (100µl/puits, 1 à 3.10⁴ cellules/ml selon la lignée considérée). Après une incubation de 24 h à 37°C dans un incubateur à 5% CO₂, le milieu a été remplacé par celui contenant le composé à tester (molécule I-1), après quoi les plaques sont incubées 48 h supplémentaires. La survie cellulaire a été évaluée par mesure de la luminescence après relargage de l'ATP dans le milieu en utilisant les solutions de lyse cellulaire, de luciférase et de luciférine contenus dans le kit ATP-lite-MTM comme cela est recommandé par le fabricant (Packard, Rungis, France). Chaque condition expérimentale a été testée au moins trois fois en sextuplet. Le tableau ci-dessous présente les données d'IC50 (exprimé en M) de la molécule I-1, en fonction des différentes lignées cellulaires testées.

| **Lignées cancéreuses** | **A549** | **BxPC3** | **LoVo** | **MCF7** | **Namalwa** | **SK-OV-3** |
|---|---|---|---|---|---|---|
| **IC50 (M)** | **8,2.10⁻¹⁰** | **4,7**.**10⁻¹⁰** | **8,1.10⁻¹¹** | **1,1.10⁻⁹** | **1,1.10⁻⁹** | **3,3**.**10**^{**-1**0} |

### Exemple 4 : Description du phénotype cellulaire (Immunofluorescence et microscopie)

Les cellules tumorales Hela (carcinome humain) ont été ensemencées pour 24 h sur des lamelles de verre de 12 mm de diamètre, placées dans des plaques de culture 24-puits (7.10³ cellules par puits).
Les cellules ont ensuite été traitées pour une nouvelle période de 24 h par la molécule I-1 aux concentrations testées. A la fin du traitement les lamelles ont été récupérées et les cellules ont été fixées et perméabilisées comme décrit précédemment (Lajoie-Mazenc I. et al., Journal of Cell Science 107, 2825-2837 (1994)). De la tubuline γ et de la tubuline α ont ensuite été marquées (2 heures - 37°C) par des anticorps primaires spécifiques dilués au 1/1000 (respectivement : anticorps polyclonal de lapin R75 (*Lajoie-Mazenc I. et al.*) ; anticorps monoclonal clone B-5-1-2, Sigma-Aldrich, France) puis révélées par les anticorps secondaires fluorescents correspondants (Alexa-568 anti-souris et Alexa-488 anti-lapin dilués au 1/1000 ; 45 min - 37°C). L'ADN a été marqué par du DAPI (0.2 µg/ml - 15 minutes - 37°C). Après les différents marquages, les lamelles ont été séchées et montées dans un liquide de montage Vectashield (AbCys, France), avant d'être observées au travers d'un microscope à épi-fluorescence (Axiovert 200M ; objectif x 63 Plan Apochromat NA, 1.4 ; ZEISS, France).

Entre 5.10⁻⁹M et 5.10⁻⁸M, les cellules interphasiques étaient dépourvues de cytosquelette microtubulaire (marquage par la tubuline α). Les centrosomes étaient souvent séparés et normalement marqués par la tubuline γ.

Avec un effet dose entre 10⁻⁹M et 5.10⁻⁸M, les cellules mitotiques ont été bloquées en prométaphase sans microtubules (marquage tubuline α). Les deux centrosomes séparés étaient normalement présents et marqués par la tubuline γ.

## Revendications

1. Composé de formule (I) suivante : 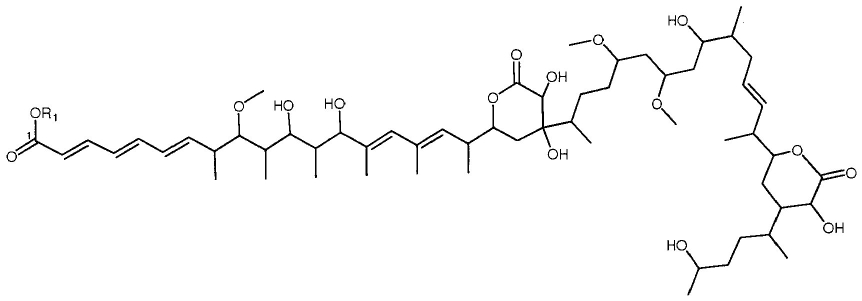 ou un sel pharmaceutiquement acceptable de celui-ci,
pour laquelle R₁ représente un atome d'hydrogène ou un groupe (C₁-C₇)alkyle.

2. Composé selon la revendication 1, **caractérisé en ce qu'**il est susceptible d'être obtenu par un procédé comprenant les étapes successives suivantes :
(i) macération d'un lyophilisat d'éponge *Hemimycale sp.* avec du chloroforme puis avec une solution hydro-éthanolique, suivie d'une filtration pour donner un filtrat, puis concentration dudit filtrat pour donner un extrait,
(ii) partition de l'extrait obtenu à l'étape (i) précédente entre de l'eau et du dichlorométhane et séparation des phases aqueuse et organique résultantes, extraction de la phase aqueuse avec de l'acétate d'éthyle et séparation des nouvelles phases aqueuse et organique résultantes, réunion des deux phases organiques ainsi obtenues et concentration pour donner un extrait dessalé,
(iii) reprise de l'extrait dessalé obtenu à l'étape (ii) précédente avec une solution hydro-méthanolique et de l'hexane, récupération et concentration de la phase méthanolique pour donner un extrait méthanolique,
(iv) isolation du composé de formule (I) avec R₁ = H à partir de l'extrait méthanolique obtenu à l'étape (iii) précédente, et
(v) éventuellement estérification de l'acide carboxylique en C-(1) pour donner un composé de formule (I) avec R₁ = (C₁-C₇)alkyle et/ou salification du composé obtenu à l'étape (iv) précédente, et isolation du composé ainsi obtenu du milieu réactionnel.

3. Composé selon la revendication 2, **caractérisé en ce que** l'éponge *Hemimycale sp*. est originaire de l'archipel de Torres du Vanuatu.

4. Composé selon l'une quelconque des revendications 1 à 3, **caractérisée en ce qu'**il est choisi parmi les composés suivants : 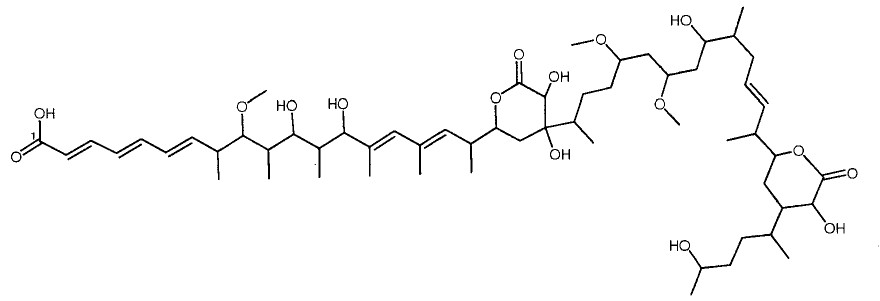 et

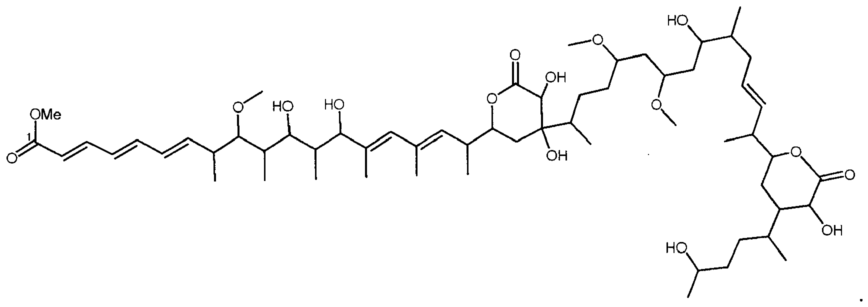

5. Composé selon l'une quelconque des revendications 1 à 4, pour son utilisation à titre de médicament.

6. Composé selon la revendication 5, pour son utilisation à titre de médicament destiné au traitement du cancer.

7. Composition pharmaceutique comprenant au moins un composé selon l'une quelconque des revendications 1 à 4 et au moins un excipient pharmaceutiquement acceptable.

8. Procédé de synthèse d'un composé selon l'une quelconque des revendications 1 à 4, comprenant les étapes successives suivantes :
(i) macération d'un lyophilisat d'éponge *Hemimycale sp.* avec du chloroforme puis avec une solution hydro-éthanolique, suivie d'une filtration pour donner un filtrat, puis concentration dudit filtrat pour donner un extrait,
(ii) partition de l'extrait obtenu à l'étape (i) précédente entre de l'eau et du dichlorométhane et séparation des phases aqueuse et organique résultantes, extraction de la phase aqueuse avec de l'acétate d'éthyle et séparation des nouvelles phases aqueuse et organique résultantes, réunion des deux phases organiques ainsi obtenues et concentration pour donner un extrait dessalé,
(iii) reprise de l'extrait dessalé obtenu à l'étape (ii) précédente avec une solution hydro-méthanolique et de l'hexane, récupération et concentration de la phase méthanolique pour donner un extrait méthanolique,
(iv) isolation du composé de formule (I) avec R₁ = H à partir de l'extrait méthanolique obtenu à l'étape (iii) précédente, et
(v) éventuellement estérification de l'acide carboxylique en C-(1) pour donner un composé de formule (I) avec R₁ = (C₁-C₇)alkyle et/ou salification du composé obtenu à l'étape (iv) précédente, et isolation du composé ainsi obtenu du milieu réactionnel.

9. Procédé de synthèse selon la revendication 8, **caractérisé en ce que** l'éponge *Hemimycale sp.* est originaire de l'archipel de Torres du Vanuatu.

10. Procédé de synthèse d'un composé selon l'une quelconque des revendications 1 à 4 avec R₁ représentant un groupe (C₁-C₇)alkyle à partir d'un composé selon l'une quelconque des revendication 1 à 4 avec R₁ = H, comprenant les étapes successives suivantes :
(a) estérification de l'acide carboxylique en C-(1) pour donner un composé de formule (I) avec R₁ = (C₁-C₇)alkyle,
(b) éventuellement salification du composé obtenu à l'étape (a) précédente, et
(c) isolation du composé obtenu à l'étape (a) ou (b) précédente du milieu réactionnel.

## Patentansprüche

1. Verbindung der folgenden Formel (I): 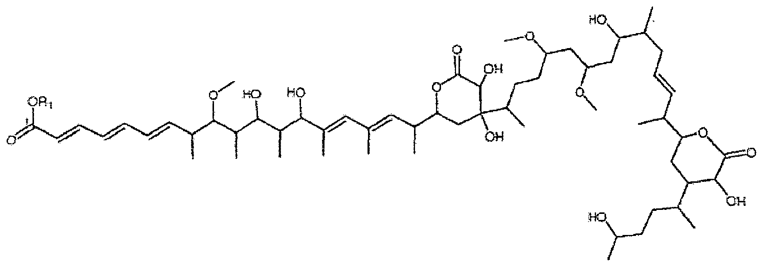 oder eines ihrer pharmazeutisch akzeptablen Salze,
wobei R₁ ein Wasserstoffatom oder eine (C₁-C₇)-Alkylgruppe repräsentiert.

2. Verbindung nach Anspruch 1, **dadurch gekennzeichnet, dass** sie durch ein Verfahren erhalten werden kann, welches die folgenden Schritte in dieser Reihenfolge umfasst:
(i) Mazerieren eines Lyophilisats eines Schwammes *Hemimycale sp.* mit Chloroform und dann mit einer wässrig-ethanolischen Lösung, gefolgt von einer Filtration, um ein Filtrat zu erhalten, und dann Konzentrieren des Filtrats, um einen Extrakt zu erhalten,
**(ii)** Aufteilen des im vorhergehenden Schritt (i) erhaltenen Extrakts zwischen Wasser und Dichlormethan und Trennen der resultierenden wässrigen Phase und organischen Phase, Extrahieren der wässrigen Phase mit Ethylacetat und Trennen der resultierenden neuen wässrigen Phase und organischen Phase, Vereinigen der zwei so erhaltenen organischen Phasen und Konzentrieren, um einen entsalzten Extrakt zu erhalten,
**(iii)** Aufnehmen des im vorhergehenden Schritt (ii) erhaltenen entsalzten Extrakts mit einer wässrig-methanolischen Lösung und Hexan, Rückgewinnen und Konzentrieren der methanolischen Phase, um einen methanolischen Extrakt zu erhalten,
**(iv)** Isolieren der Verbindung der Formel (I) mit R₁ = H aus dem im vorhergehenden Schritt (iii) erhaltenen methanolischen Extrakt, und
**(v)** ggf. Verestern der Carbonsäure an C-(1), um eine Verbindung der Formel (I) mit R₁ = (C₁-C₇)-Alkyl zu erhalten, und/oder Umwandeln der im vorhergehenden Schritt (iv) erhaltenen Verbindung in ein Salz, und Isolieren der so erhaltenen Verbindung aus dem Reaktionsmedium.

3. Verbindung nach Anspruch 2, **dadurch gekennzeichnet, dass** der Schwamm *Hemimycale sp.* von den Torres-Inseln von Vanuatu stammt.

4. Verbindung nach einem der Ansprüche 1 bis 3, **dadurch gekennzeichnet, dass** sie ausgewählt ist aus den folgenden Verbindungen: 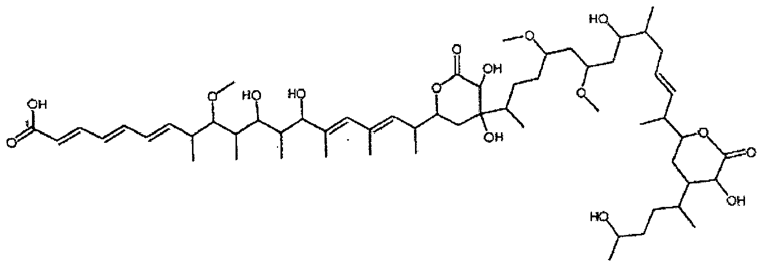 und

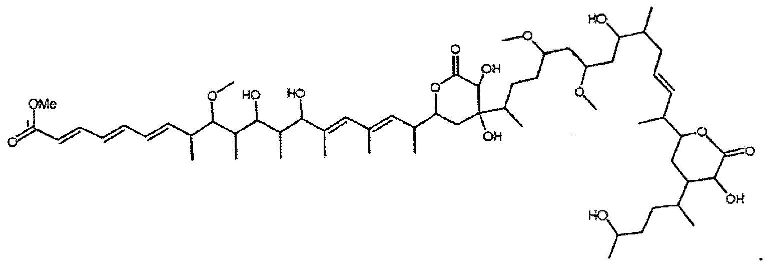

5. Verbindung nach einem der Ansprüche 1 bis 4 zur Verwendung als Medikament.

6. Verbindung nach Anspruch 5 zur Verwendung als Medikament, das zur Behandlung von Krebs bestimmt ist.

7. Pharmazeutische Zusammensetzung, umfassend mindestens eine Verbindung nach einem der Ansprüche 1 bis 4 und mindestens einen pharmazeutisch akzeptablen Hilfsstoff.

8. Verfahren zur Synthese einer Verbindung nach einem der Ansprüche 1 bis 4, umfassend die folgenden Schritte in dieser Reihenfolge:
**(i)** Mazerieren eines Lyophilisats eines Schwammes *Hemimycale sp.* mit Chloroform und dann mit einer wässrig-ethanolischen Lösung, gefolgt von einer Filtration, um ein Filtrat zu erhalten, und dann Konzentrieren des Filtrats, um einen Extrakt zu erhalten,
**(ii)** Aufteilen des im vorhergehenden Schritt (i) erhaltenen Extrakts zwischen Wasser und Dichlormethan und Trennen der resultierenden wässrigen Phase und organischen Phase, Extrahieren der wässrigen Phase mit Ethylacetat und Trennen der resultierenden neuen wässrigen Phase und organischen Phase, Vereinigen der zwei so erhaltenen organischen Phasen und Konzentrieren, um einen entsalzten Extrakt zu erhalten,
**(iii)** Aufnehmen des im vorhergehenden Schritt (ii) erhaltenen entsalzten Extrakts mit einer wässrig-methanolischen Lösung und Hexan, Rückgewinnen und Konzentrieren der methanolischen Phase, um einen methanolischen Extrakt zu erhalten,
**(iv)** Isolieren der Verbindung der Formel (I) mit R₁ = H aus dem im vorhergehenden Schritt (iii) erhaltenen methanolischen Extrakt, und
**(v)** ggf. Verestern der Carbonsäure an C-(1), um eine Verbindung der Formel (I) mit R₁ = (C₁-C₇)-Alkyl zu erhalten, und/oder Umwandeln der im vorhergehenden Schritt (iv) erhaltenen Verbindung in ein Salz, und Isolieren der so erhaltenen Verbindung aus dem Reaktionsmedium.

9. Verfahren zur Synthese nach Anspruch 8, **dadurch gekennzeichnet, dass** der Schwamm *Hemimycale sp.* von den Torres-Inseln von Vanuatu stammt.

10. Verfahren zur Synthese einer Verbindung nach einem der Ansprüche 1 bis 4, bei der R₁ eine (C₁-C₇)-Alkylgruppe repräsentiert, aus einer Verbindung nach einem der Ansprüche 1 bis 4 mit R₁ = H, umfassend die folgenden Schritte in dieser Reihenfolge:
(a) Verestern der Carbonsäure an C-(1), um eine Verbindung der Formel (I) mit R₁ = (C₁-C₇)-Alkyl zu erhalten,
(b) ggf. Umwandeln der im vorhergehenden Schritt (a) erhaltenen Verbindung in ein Salz, und
(c) Isolieren der im vorhergehenden Schritt (a) oder (b) erhaltenen Verbindung aus dem Reaktionsmedium.

## Claims

1. A compound of following formula (I): 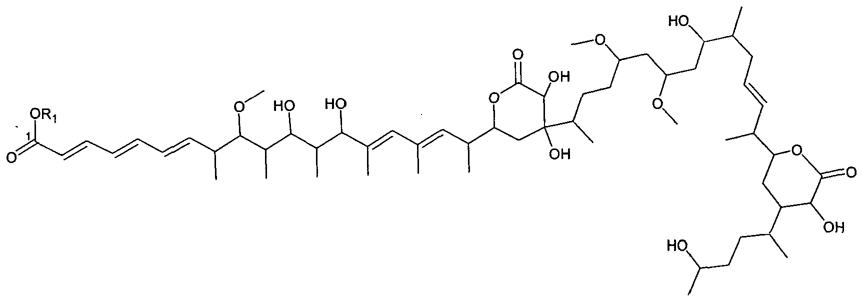 or a pharmaceutically acceptable salt thereof, where R₁ is a hydrogen atom or a (C₁-C₇) alkyl group.

2. The compound according to claim 1, **characterised in that** it is obtainable by a method comprising the following successive steps:
(i) maceration of a lyophilisate of *Hemimycale sp*. sponge with chloroform then with a hydro-ethanolic solution, followed by filtration to give a filtrate, then concentration of said filtrate to give an extract,
(ii) partition of the extract obtained at the preceding step (i) between water and dichloromethane and separation of the resulting aqueous and organic phases, extraction of the aqueous phase with ethyl acetate and separation of the new resulting aqueous and organic phases, combining the two organic phases thereby obtained and concentration to give a desalinated extract,
(iii) taking up the desalinated extract obtained at the preceding step (ii) with a hydro-methanolic solution and hexane, recovery and concentration of the methanolic phase to give a methanolic extract,
(iv) isolation of the compound of formula (I) with R₁ = H from the methanolic extract obtained at the preceding step (iii), and
(v) optionally esterification of carboxylic acid at C-(1) to give a compound of formula (I) with R₁ = (C₁-C₇) alkyl and/or salification of the compound obtained at the preceding step (iv), and isolation of the compound thereby obtained from the reaction mixture.

3. The compound according to claim 2, **characterised in that** the sponge *Hemimycale sp.* originates from the Torres archipelago, Vanuatu.

4. The compound according to any of claims 1 to 3, **characterised in that** it is selected from the following compounds: 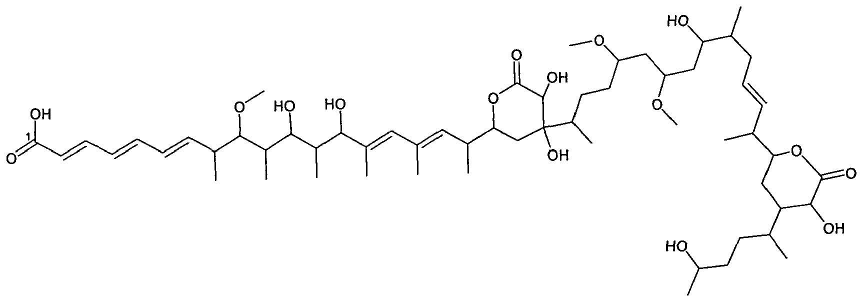 and

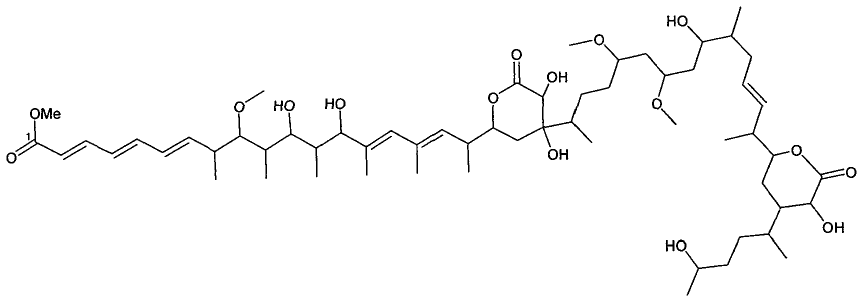

5. A compound according to any of claims 1 to 4, for its use as medicine.

6. The compound according to claim 5, for its use as medicine intended for the treatment of cancer.

7. A pharmaceutical composition comprising at least one compound according to any of claims 1 to 4 and at least one pharmaceutically acceptable excipient.

8. A method of synthesizing a compound according to any of claims 1 to 4, comprising the following successive steps:
(i) maceration of lyophilisate of *Hemimycale sp.* sponge with chloroform then with a hydro-ethanolic solution, followed by filtration to give a filtrate, then concentration of said filtrate to give an extract,
(ii) partition of the extract obtained at the preceding step (i) between water and dichloromethane and separation of the resulting aqueous and organic phases, extraction of the aqueous phase with ethyl acetate and separation of the new resulting aqueous and organic phases, combination of the two organic phases thereby obtained and concentration to give a desalinated extract,
(iii) taking up the desalinated extract obtained at the preceding step (ii) with a hydro-methanolic solution and hexane, recovery and concentration of the methanolic phase to give a methanolic extract,
(iv) isolation of the compound of formula (I) with R₁ = H from the methanolic extract obtained at the preceding step (iii), and
(v) optionally esterification of the carboxylic acid at C-(1) to give a compound of formula (I) with R₁ = (C₁-C₇) alkyl and/or salification of the compound obtained at the preceding step (iv), and isolation of the compound thereby obtained from the reaction mixture.

9. The method of synthesizing according to claim 8, **characterised in that** the sponge *Hemimycale sp.* originates from the Torres archipelago, Vanuatu.

10. A method of synthesizing a compound according to any of claims 1 to 4 with R₁ representing a (C₁-C₇) alkyl group from a compound according to any of claims 1 to 4 with R₁ = H, comprising the following successive steps:
(a) esterification of the carboxylic acid at C-(1) to give a compound of formula (I) with R₁ = (C₁-C₇) alkyl,
(b) optionally salification of the compound obtained at the preceding step (a), and
(c) isolation of the compound obtained at the preceding step (a) or (b) from the reaction mixture.
